# EUROPEAN PATENT SPECIFICATION

(11) **EP 1 568 306 B1**
(45) Date of publication and mention of the grant of the patent: **30.11.2011**
(21) Application number: 05002698.8
(22) Date of filing: 09.02.2005
(51) Int. Cl.: A61B 1/018, A61M 25/01

(54) **Endoscope**
Endoskop
Endoscope

(30) Priority: 26.02.2004 JP 2004051365
(43) Date of publication of application: 31.08.2005
(73) Proprietor: Olympus Corporation, Shibuya-ku, Tokyo (JP)
(72) Inventor: Iizuka, Shuhei, Tachikawa-shi Tokyo (JP); Nagase, Toru, Tachikawa-shi Tokyo (JP); Okada, Tsutomu, Tachikawa-shi Tokyo (JP); Suzuki, Keita, Kokubunji-shi Tokyo 185-0035 (JP)
(74) Representative: von Hellfeld, Axel

(56) References cited:
- WO-A-96/01592
- JP-A- 57 190 541
- US-A- 4 976 697
- US-A- 5 931 833
- US-B1- 6 171 234
- PATENT ABSTRACTS OF JAPAN vol. 1997, no. 10, 31 October 1997 (1997-10-31) & JP 09 140663 A (OLYMPUS OPTICAL CO LTD), 3 June 1997 (1997-06-03)
- PATENT ABSTRACTS OF JAPAN vol. 1997, no. 05, 30 May 1997 (1997-05-30) & JP 09 000492 A (OLYMPUS OPTICAL CO LTD), 7 January 1997 (1997-01-07)

## Description

### BACKGROUND OF THE INVENTION

### 1. Field of the Invention

The present invention relates to an endoscope(for medical and industrial use). More specifically, the present invention relates to a medical endoscope used for being inserted into a body of a human or an animal, or for industrial applications. The features of the preamble of claim 1 are known from JP 09-140663.

### 2. Description of the Related Art

Performing inspection or observation by inserting into a cavity an endoscope insertion part (adapted for medical or industrial uses) is known. The endoscope has a flexible insertion portion, and an image-capturing means is provided at the distal end thereof, so that observation in the cavity is enabled. In many cases, the endoscope includes a treatment tool channel which penetrates from the distal end to the proximal end thereof (out of the body), and various treatments can be performed by inserting a treatment tool for the endoscope, such as a forceps, into the treatment tool channel.

US-A-5,931,833 relates to an endoscopic accessory and containment system for use with a flexible or rigid endoscopic. The system includes an accessory and a containment enclosure, wherein the accessory has an elongated, flexible and axially-rigid shaft shaped to fit into the biopsy channel of the endoscope and a tool connected to the distal end of the shaft. The enclosure is adapted to contain at least a major portion of the shaft and is remote from the endoscope and has at least one aperture through which the distal end of the shaft extends. An instrument deploying mechanism connected to the enclosure and to the shaft has a withdrawing mechanism that withdraws the shaft into the enclosure through the aperture. A control mechanism, such as control wheels, for controlling the tool is attached to the proximal end of the shaft. Here, the tool, such as a forceps, can be controlled by operating the control mechanism and by extending or withdrawing the shaft relative to the endoscopic tube.

JP 09-140663 discloses an endoscope with a motor which drives two rollers for inserting a treatment tool into a lumen of the endoscope. A cam mechanism can be operated to change the position of one of the rollers such that the distance between the rollers when the operator desires to terminate the motor-driven advancement of the treatment tool, is increased and then the operator can manually move the treatment tool by pushing or pulling the treatment tool.

WO 1996/001592 discloses an endoscope having two rollers for inserting/withdrawing a treatment tool. One of the rollers can be operated by an operator to drive the treatment tool in a forward or backward direction.

US 4,976,697 discloses a means to be operated by an operator for feeding a catheter periodically forward and backward within a lumen to allow selective feeding of fluid through the catheter.

The technology disclosed in JP-A-57-117823 is configured so that when inserting or removing the treatment tool into/from the treatment tool channel, such insertion and withdrawal of the treatment tool can be carried out automatically. More specifically, a micro-motor and two drums that can be rotated by the micro-motor are provided in the endoscope, and the treatment tool is tightly held between the outer peripheral surfaces of the drums. When an operator controls the micro-motor to allow the respective drums to rotate in an appropriate directions, the treatment tool held between the drums can be inserted or withdrawn.

In an endoscope of this type, the treatment tool can be inserted and withdrawn into/from the treatment tool channel automatically. However, it is difficult to precisely position a treatment section, i.e., a treatment administering end, provided at the distal end of the treatment tool at the intended position in the cavity by moving it in the fore-and-aft direction once the treatment tool is in the tool channel of the endoscope. This is because the treatment tool end needs to be moved over a very short distance from the tip end of the channel, after it has been completely inserted into the endoscope. This distance is very short compared to the distance the tool must travel in the endoscope channel. Therefore, precise, i.e., fine, control of the treatment tool's movements is very difficult with conventional methods.

To solve this problem, it has been proposed to provide a speed reducing mechanism for changing the speed of rotation of the drums. However, such a speed reducing mechanism has a complex structure, which increases significantly the cost of the endoscope.

In view of these circumstances, it is an object of the present invention to provide an endoscope capable of inserting and removing the treatment tool, and in which the treatment section or the end of the tool can be more simply and accurately moved in the fore-and-aft direction in a cavity.

### BRIEF SUMMARY OF THE INVENTION

The problem is solved by the features of claim 1.

The present invention provides an endoscope having a treatment tool insertion/withdrawal mechanism for inserting and removing the treatment tool with respect to a treatment tool channel, in which a fine-adjustment mechanism for fine-adjusting the position of the distal portion of the treatment tool with respect to the distal end of the treatment tool channel is provided.

According to the present invention, since the position of the distal portion of the treatment tool can be fine-adjusted, it is easy to accurately position the distal portion of the treatment tool with respect to the subject to be treated. The treatment tool insertion/withdrawal mechanism is preferably configured to move the treatment tool by means of motive power of a motor.

The term "insertion" means to insert the treatment tool into the treatment tool channel to reach the distal end, i.e., the distal portion of the endoscope. The term "withdrawal" means to withdraw the treatment tool entirely, or almost entirely, from the treatment tool channel.

The fine-adjustment mechanism is disposed to be partly exposed so that an operator can manually operate it.

Such an endoscope can be operated manually with the fingers of the operator working the accessible controls of the fine-adjustment mechanism. The treatment tool can be moved in the fore-and-aft direction according to the amount of operation, e.g., movement, of the fine-adjustment mechanism, which is operated by hand. Therefore, fine adjustment can be made easily.

The fine-adjustment mechanism is a mechanism for rotating a reel for storing the proximal portion of the treatment tool, which tool is inserted and withdrawn by the treatment tool insertion/withdrawal mechanism.

This endoscope has a structure that winds the treatment tool on the reel, and the treatment tool is moved in the fore-and-aft direction by rotating the reel. The treatment tool can be stored compactly by the reel, by being wound thereon. Since the fine-adjustment mechanism can be used as the reel and as a storage section, the structure is simple.

The treatment tool insertion/withdrawal mechanism includes a roller for driving the treatment tool along the axis thereof, and is characterized in that the fine-adjustment mechanism alternatively is a means that rotates the roller.

Preferable structure in this case is such that, for example, the treatment tool insertion/withdrawal mechanism rotates the roller electrically, and the fine-adjustment mechanism rotates the roller manually.

Preferably, the fine-adjustment mechanism is provided on the treatment tool insertion/withdrawal mechanism.

Since the endoscope includes the fine-adjustment mechanism on the treatment tool insertion/withdrawal mechanism, the structure of the device is simplified.

According to the present invention, since the fine-adjustment mechanism is provided, the position of the treatment tool can be fine-adjusted in a state in which the treatment tool is inserted into the treatment tool channel using the treatment tool insertion/withdrawal mechanism.

### BRIEF DESCRIPTION OF THE SEVERAL VIEWS OF THE DRAWINGS

These and other features, aspects, and advantages of the apparatus and methods of the present invention will become better understood with regard to the following description, appended claims, and accompanying drawings where:
Fig. 1 is a general view showing a structure of an endoscope according to an embodiment of the present invention;
Fig. 2 is a drawing viewed in the direction indicated by an arrow in Fig. 1;
Fig. 3 is a cross-sectional view of a structure of a treatment tool insertion/withdrawal device and a storage device showing a state in which a treatment tool is inserted;
Fig. 4 is a cross-sectional view of Fig. 3;
Fig. 5 is a cross-sectional view of a structure of the treatment tool insertion/withdrawal device and the storage device, showing a state in which the treatment tool is withdrawn;
Fig. 6 is a cross-sectional view of a structure of the treatment tool insertion/withdrawal device and the storage device, showing a state in which the treatment tool is inserted; and
Fig. 7 is a cross-sectional view of a structure of the treatment tool insertion/withdrawal device and the storage device, showing a state in which the treatment tool is inserted.

### DETAILED DESCRIPTION OF THE PREFERRED EMBODIMENTS

Preferred embodiments of the invention are described below with reference to the accompanying drawings.

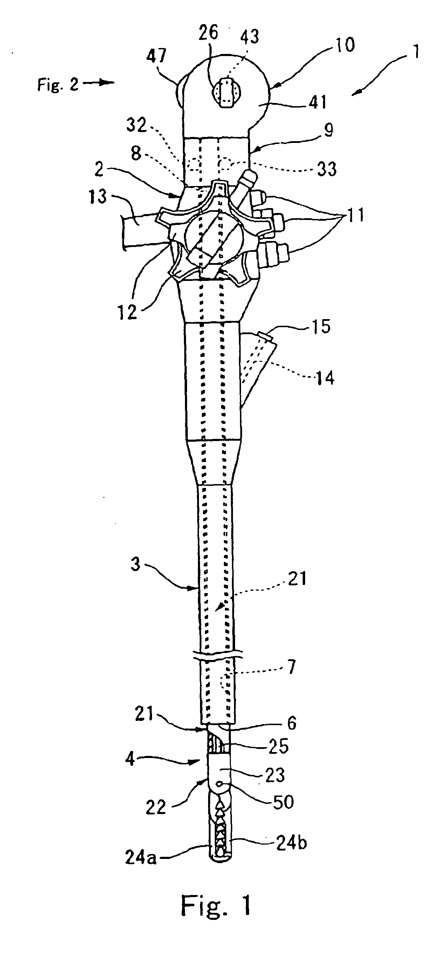

Fig. 1 shows a general structure of a medical endoscope according to a first embodiment.

As shown in Fig. 1, an endoscope 1 has a final operating element 2 to be operated by an operator such as a practitioner, and a flexible insertion portion 3 to be inserted into a body cavity provided at the distal end of the final operating element 2. The insertion portion 3 is provided with an image-capturing device, an optical system (not shown) for illumination, and so on, and a distal opening 6 of a treatment tool channel 7 for receiving a treatment tool 4 to be inserted therethrough is provided at the distal end thereof. The treatment tool channel 7 penetrates the endoscope 1 from the distal end of the insertion portion 3 to the proximal end of the final operating element 2, and an insertion port 8 for inserting the treatment tool 4 is formed on the proximal side of the final operating element 2. A storage device 10 for the treatment tool 4 is mounted to the proximal end of the final operating element 2 via a treatment tool insertion/withdrawal device 9.

A switch 11 for setting parameters, such as a changeover of illumination, and a knob 12 for changing the direction of the distal end of the insertion portion 3 are provided on the outer peripheral surface of the final operating element 2. A universal cable 13 to be connected to a control device (not shown) is connected to the side portion of the final operating element 2. Below the side portion where the switch 11 is provided, there is formed an insertion port 15 of another treatment tool channel 14. The treatment tool channel 14 and the insertion port 15 are not essential components.

Fig. 1 shows grip forceps as an example of the treatment tool 4. Such the treatment tool 4 has a treatment tool insertion portion 21 to be inserted into the treatment tool channel 7. The treatment tool insertion portion 21 is provided with a flexible and tightly-wound sheath. The treatment tool insertion portion 21 is provided with a treatment section 22 at the distal end thereof. The treatment section 22 includes a distal end cover (supporting member) 23 fixed to the distal end of the treatment tool insertion portion 21, a pair of grip members 24a, 24b rotatably supported by the distal end cover 23, and a link mechanism (not shown) for rotating the grip members 24a, 24b. The link mechanism is connected to the grip members 24a, 24b at one end and is connected to the distal end of an operating wire 25 at the other end. The operating wire 25 is inserted into the treatment tool insertion portion 21, and the operating wire 25 and the treatment tool insertion portion 21 pass through the treatment tool channel 7, and are drawn out from the insertion port 8 at the proximal side, inserted through the treatment tool insertion/withdrawal device 9, and are wound in the storage device 10. The proximal end of the operating wire 25 is attached to a final operating element 26 provided outside the storage device 10.

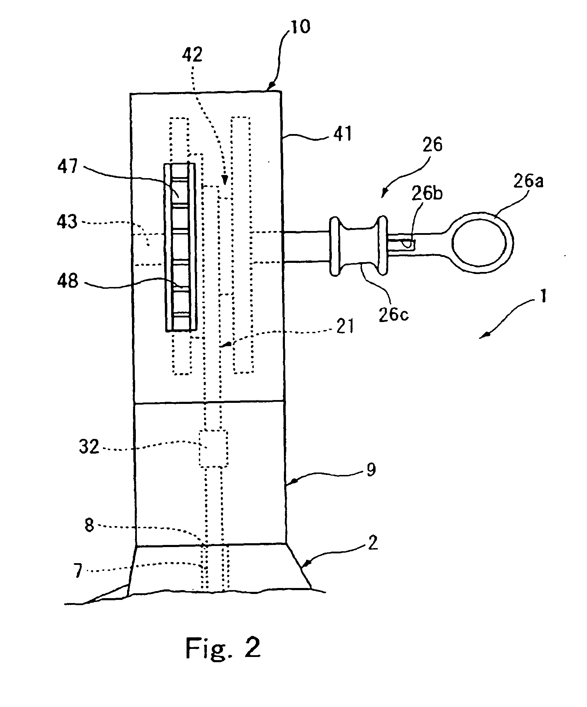

Fig. 2 is a drawing of the upper portion of Fig. 1 viewed in the direction indicated by the arrow which bears the legend "Fig. 2" in Fig. 1. As shown in Fig. 2, the final operating element 26 is connected at one end to the side portion of the storage device 10, and extends substantially perpendicularly therefrom. A finger ring 26a is provided on the other end of the final operating element 26. The final operating element 26 is formed with a slit 26b so as to extend in parallel with the lengthwise direction thereof, and a slider 26c is slidably mounted thereto. The proximal end of the operating wire 25 is inserted into the main body of the final operating element 26 so as to be capable of moving in the fore-and-aft direction freely, and is fixed to the slider 26c.

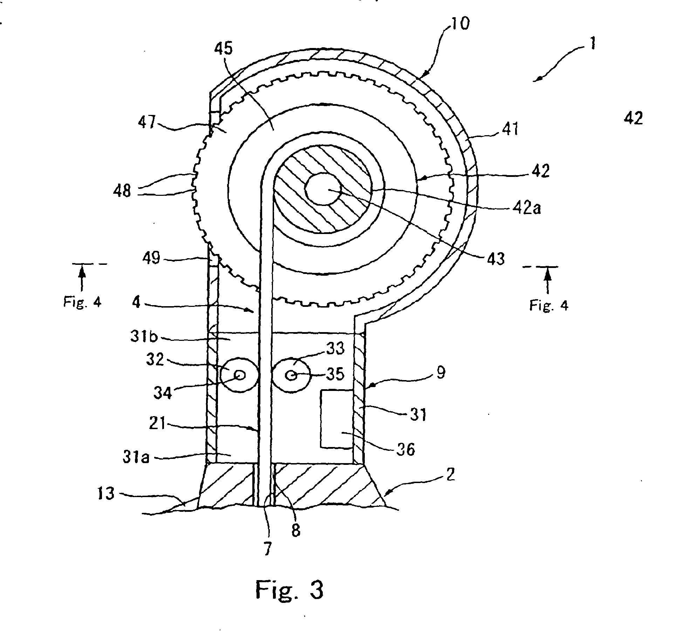

Fig. 3 is a cross-sectional view showing structures of the treatment tool insertion/withdrawal device 9 and the storage device 10. As shown in Fig. 3, the treatment tool insertion/withdrawal device 9 includes a cover 31 fixed to the proximal side of the final operating element 2, and openings 31a, 31b, for receiving the treatment tool insertion portion 21, disposed coaxially with the treatment tool channel 7. The cover 31 is fixed to the final operating element 2 so as to cover the insertion port 8 of the treatment tool channel 7 by the opening 31a. The cover 31 is also provided with a pair of rollers 32, 33, mounted to be freely rotatable.

The respective rollers 32, 33 are disposed so that the outer peripheral surfaces thereof are in tight, pressure contact with the treatment tool insertion portion 21 and so that a straight line connecting revolving shafts 34, 35 of the respective rollers 32, 33 is substantially orthogonal to the direction of insertion/withdrawal of the treatment tool 4. The respective rollers 32, 33 are connected to a motor 36 which comprises the drive source, via a transmission mechanism (not shown) including a gear or the like. The transmission mechanism is configured to rotate the roller 33 in the reverse direction (counterclockwise in Fig. 3) to rotate the roller 32 in the normal direction (clockwise in Fig. 3), and to rotate the roller 33 in the normal direction to rotate the roller 32 in the reverse direction. The outer peripheral surfaces of the rollers 32, 33 preferably have a rough surface to prevent slippage.

The motor 36 is operated by a switch (not shown) provided on the cover 31. The respective roller 32, 33 can rotate freely when the switch of the motor 36 is turned off, that is, when the power supply of the treatment tool insertion/withdrawal device 9 is cut. For example, a gear interposed between the gear on the side of the rollers 32, 33 and the motor 36 is fixed to a movable core of a solenoid, so that the power is applied to a coil of the solenoid when the power of the treatment tool insertion/withdrawal device 9 is turned on. In this case, when the power is turned on, the movable core moves to cause the gears to engage, and the rotation from the motor 36 is transmitted to the respective rollers 32, 33. In contrast, when the power supply is cut, the movable core moves to cause the gears to be disengaged, and hence the motor 36 and the respective rollers 32, 33 are mechanically disconnected. Therefore, the rollers 32, 33 are released from any static torque of the motor 36. In order to realize such an action, the switch of the treatment tool insertion/withdrawal device 9 can be switched to at least three positions; a position where the power supply is cut, a position to rotate the rollers 32, 33 in the direction of insertion of the treatment tool, and a position to rotate the rollers 32, 33 in the direction of withdrawal of the treatment tool 4.

The storage device 10 includes a reel cover 41 to be attached so as to cover the opening 31b of the treatment tool insertion/withdrawal device 9, and a reel 42 rotatably supported in the reel cover 41.

A revolving shaft 43 of the reel 42 is disposed at a position offset from the axis of the treatment tool channel 7 in a direction substantially orthogonal to the axis. The revolving shaft 43 is offset with respect to the treatment tool channel 7 to enable smooth delivering of the treatment tool 4 wound on the reel 42 to the treatment tool insertion/withdrawal device 9. In this embodiment, the revolving shaft 43 is disposed at a position opposite from the universal cable 13 with respect to the axis of the treatment tool channel 7.

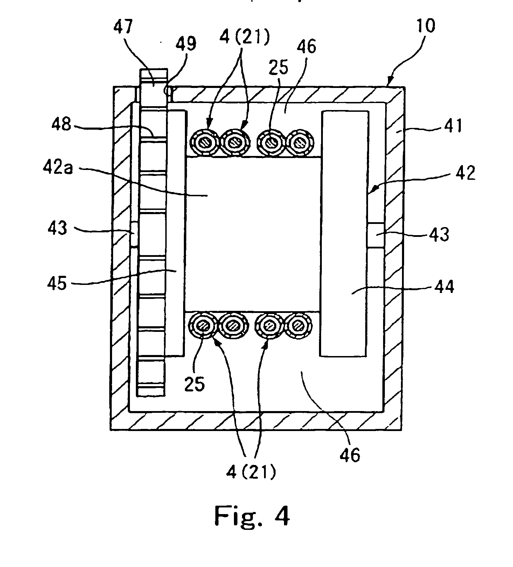

Fig. 4 is a cross-sectional view of Fig. 3. As shown in Fig. 4, edges of the reel 42 in the longitudinal direction of the revolving shaft 43 have increased diameters and form flanges 44, 45. The treatment tool insertion portion 21 of the treatment tool 4 is wound on an outer peripheral surface 42a of the reel 42, between the two flanges 44, 45. A space 46 defined by the outer peripheral surface 42a and the reel cover 41 forms the storage space 46 for winding therein the treatment tool 4. The size of the space 46 is selected to accommodate the particular thickness and length of the treatment tool 4 to be wound on the reel 42.

The flange 45 of the reel 42 is further increased in diameter at the outer edge thereof, and the enlarged portion forms a dial member 47 constituting a fine-adjustment mechanism. The dial member 47 is formed with a plurality of grooves 48 at predetermined intervals entirely along the outer peripheral surface thereof. Part of the dial member 47 projects outwardly from an opening 49 formed on the reel cover 41. The opening 49 is provided on the side of the universal cable 13 with respect to the axis of the treatment tool channel 7, so that the operator can place his/her thumb on the dial member 47 when he/she holds the final operating element 2 with his/her hand.

Although not shown in the drawing, the proximal end of the operating wire 25 of the treatment tool 4 wound on the reel 42 is pulled out from the revolving shaft 43, and the proximal end thereof is provided with a slider 26c (Fig. 2) on which the operator or an assistant of the operator places his/her finger.

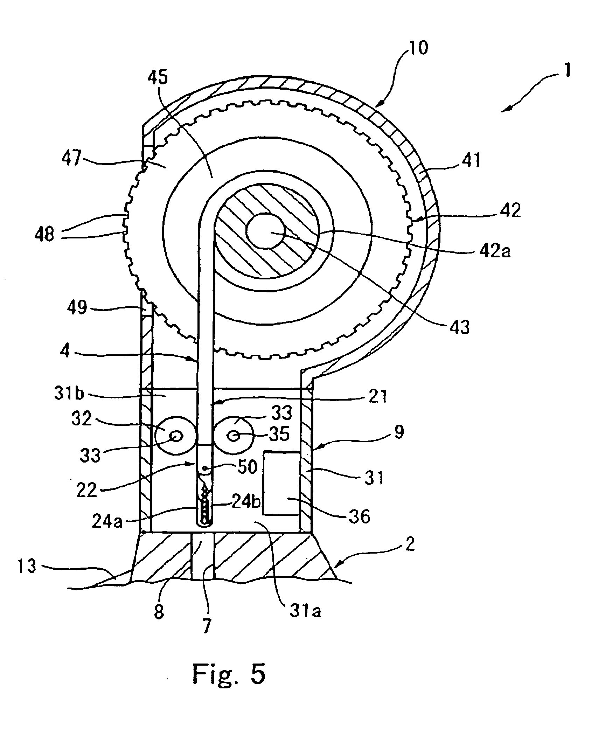

The operation of this embodiment is described below. As shown in Fig. 5, in the initial state, the treatment tool 4 is wound on the reel 42, and the treatment section 22 at the distal end of the tool 4 is positioned just slightly beyond the rollers 32, 33 of the treatment tool insertion/withdrawal device 9.

In use, the operator first inserts the insertion portion 3 of the endoscope 1 shown in Fig. 1 into a body cavity of a patient. The operator operates the knob 12 of the final operating element 2 or the like to change the direction of the distal end of the insertion portion 3 to position it close to the body part to be treated in the body cavity.

Then, the operator inserts the treatment tool 4 which has been wound inside the storage device 10 into the treatment tool channel 7. More specifically, the operator operates the switch of the treatment tool insertion/withdrawal device 9 to rotate the motor 36 in the direction of insertion. Accordingly, the rollers 32, 33 rotate to push the treatment tool insertion portion 21 clamped between the rollers 32, 33 toward the distal end of the endoscope. To do so, the roller 32 rotates in one direction (clockwise), while the roller 33 rotates in the opposite direction. The treatment tool 4 is thereby inserted from the insertion port 8 into the treatment tool channel 7 by the rotation of the rollers 32, 33, and the treatment tool insertion portion 21 is pulled out from the reel 42 to the extent of the amount of insertion thereof. During this phase of the operation, the reel 42 is allowed to rotate freely with respect to the reel cover 41, and the treatment tool insertion portion 21 wound on the reel 42 is delivered quickly.

When the distal end of the treatment tool 4, that is, the treatment section 22 projects from the distal opening 6 of the treatment tool channel 7 toward the desired body part in the body cavity, the operator operates the switch and cuts the power supply to the treatment tool insertion/withdrawal device 9. Accordingly, insertion of the treatment tool 4 is stopped.

Subsequently, the operator operates the slider 26c (see Fig. 2) of the final operating element 26 extending vertically from the storage device 10 and inserts the operating wire 25 (by moving it forward). Accordingly, the link member connected to the distal end of the operating wire 25 moves in the predetermined direction, and the pair of grip members 24a, 24b open about a revolving shaft 50.

Then, the operator places his/her thumb on the dial member 47 exposed from the reel cover 41 of the storage device 10 and rotates the dial member 47, that is, the reel 42 in the direction to precisely position the treatment tool 4 (counterclockwise in Fig. 3). The reel 42 rotates in direct relation to the amount of turning of the dial member 47, and the treatment tool 4 is delivered toward the treatment tool channel 7 correspondingly. As described above, since the respective rollers 32, 33 of the treatment tool insertion/withdrawal device 9 can be rotated freely when the power supply is cut, the treatment tool insertion portion 21 moves in the treatment tool insertion/withdrawal device 9 and the treatment tool channel 7 toward the front and the treatment section 22 (see Fig. 1) moves forward correspondingly.

The operator is able to move the treatment tool 4 forward in small increments by operating the dial. And when the open grip members 24a, 24b become pressed against the desired body part, the operating wire 25 is moved back. In response, the link member connected to the distal end of the operating wire 25 moves in a direction opposite from the direction described above, and the grip members 24a, 24b close about the revolving shaft and clamp the body part.

In this state, the operator rotates the dial member 47 in a direction opposite from the direction of delivery, that is, in the winding direction. The reel 42 then winds the treatment tool 4 according to the turning amount of the dial member 47, and the treatment tool insertion portion 21 and the treatment section 22 are moved backward correspondingly. At this time the subject portion, i.e., the body tissue clamped by the grip members 24a, 24b, is pulled toward the endoscope 1.

After having completed a predetermined treatment using other treatment tools or the like in a state in which the subject portion is being pulled, the operator works the operating wire 25 to open the grip members 24a, 24b, and the subject portion is released.

When removing the treatment tool 4 from the treatment tool channel 7, the operator operates the operating wire 25 and closes the grip members 24a, 24b. Subsequently, the operator lets go of the dial member 47, and operates the switch of the treatment tool insertion/withdrawal device 9, which causes the motor 36 to rotate in the direction of withdrawal. Accordingly, the rollers 32, 33 rotate in the direction in which the treatment tool 4 is pulled out from the treatment tool channel 7. More specifically, the roller 32 rotates in the reverse direction, while the roller 33 rotates in the forward direction. By the rotation of the rollers 32, 33, the treatment tool 4 is pulled from the treatment tool channel 7 through the treatment tool insertion/withdrawal device 9 and into the storage device 10. In the storage device 10, the treatment tool insertion portion 21 is pushed between the reel 42, the reel cover 41, and hence the reel 42 rotates in the winding direction (clockwise in Fig. 3), whereby the treatment tool 4 is wound by the reel 42.

When the treatment tool 4 has been wound until the distal end (treatment section 22) of the treatment tool 4 has been pulled out from the treatment tool channel 7, the switch is activated to cut the power supply to the treatment tool insertion/withdrawal device 9. The withdrawal of the treatment tool 4 is then terminated.

According to the first embodiment, since a portion of the reel 42 is exteriorly accessible to enable manual rotation of the dial member 47, the endoscope 1 in which the treatment tool 4 can be inserted and withdrawn electrically, permits the treatment section 22 projecting from the distal end of the endoscope 1 to be manually moved in the fore-and-aft direction with respect to the distal end of the endoscope 1 (or the subject portion). Therefore, the position of the treatment section 22 can be fine-adjusted to a position suitable for treatment and the treatment tool 4 can be pulled back.

Since part of the reel 42 is configured to be the fine-adjustment mechanism so that the treatment tool 4 can be moved directly in the fore-and-aft direction, more reflexive operation thereof is enabled, which permits use by technicians. In addition, since the reel 42 is also provided with the fine-adjustment mechanism, its structure is simpler, enabling size and cost reductions as well.

The treatment tool 4 may be any type of treatment tool as long as it can be inserted into the treatment tool channel 7 of the endoscope 1 for such uses as grip forceps, basket-type forceps, a snare, an opaque tube, and so on.

The distal end cover 23 of the treatment section 22 may be provided with a flange. Such a flange will abut the rollers 32, 33 when the treatment tool 4 is withdrawn, and prevent the treatment tool 4 from being further wound in by the reel 42. Thus, the flange functions as means for controlling the amount of withdrawal of the treatment tool 4.

It is also possible to form at least part of the cover 31 of the treatment tool insertion/withdrawal device 9 of a transparent member so that the state and amount of withdrawal of the treatment tool 4 can be checked visually.

Referring to the drawings, a second embodiment of the present invention will be described. The parts which are in the first embodiment are represented by the same reference numerals and the description thereof is omitted.

This embodiment is characterized in that the fine-adjustment mechanism is provided on the treatment tool insertion/withdrawal device.

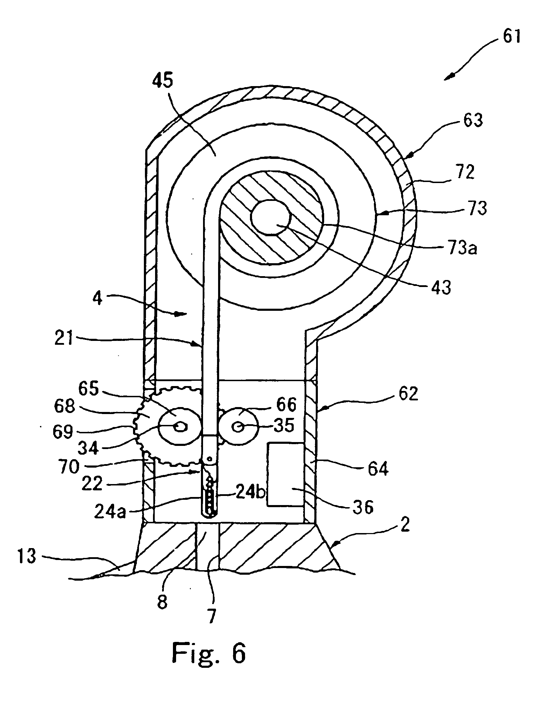

As shown in Fig. 6, an endoscope 61 includes a treatment tool insertion/withdrawal device 62 on the proximal side of the final operating element 2 so as to cover the insertion port 8, and a storage device 63 is provided at the proximal end of the treatment tool insertion/withdrawal device 62.

The treatment tool insertion/withdrawal device 62 includes two rollers 65, 66 rotatably mounted in a cover 64. The two rollers 65, 66 are disposed so as to hold the treatment tool insertion portion 21 by the outer peripheries thereof. The revolving shaft 34 of the roller 65 and the revolving shaft 35 of the roller 66 are disposed so as to extend substantially orthogonally to the direction of insertion and withdrawal of the treatment tool insertion portion 21. The motor 36 is connected to the respective rollers 65, 66 via a transmission mechanism (not shown) including a gear or the like. The transmission mechanism is configured to rotate the roller 66 in reverse direction to the normal rotation of the roller 65, and vice versa, to rotate the roller 66 in the normal direction to rotate the roller 65 in the reverse direction. The transmission mechanism is configured so that the respective rollers 65, 66 and the motor 36 become mechanically disconnected when the power supply of the treatment tool insertion/withdrawal device 62 is cut.

One of the round edge parts of the roller 65 has a larger diameter and forms a dial member 68, which comprises a fine-adjustment mechanism. The outer peripheral surface of the dial member 68 is formed with a plurality of grooves 69 at predetermined intervals entirely along the outer peripheral surface thereof. The dial member 68 partly projects outwardly from an opening 70 formed on the cover 64. The opening 70 is formed on the side wall of the treatment tool insertion/withdrawal device 62 on the side where the universal cable 13 extends. The shape of the cover 64 is the same as the cover 31 (see Fig. 3) in the first embodiment other than for the fact that the opening 70 is provided.

The storage device 63 includes a reel 73 rotatably supported by a reel cover 72. The reel 73 is the same as the reel 42 in the first embodiment (see Fig. 4) except that no dial member is provided. In other words, the reel 73 includes the flange 44 (see Fig. 4) and the flange 45 on both ends, and the outer diameters of the respective flanges 44, 45 are substantially the same. The revolving shaft 43 of the reel 73 is disposed on the opposite side from the universal cable 13 with respect to the axis of the treatment tool channel 7. Then, the treatment tool 4 is wound on an outer peripheral surface 73a defined between the flange 44 and the flange 45.

The operation of this embodiment is as follows.

When inserting the treatment tool 4, the operator operates the switch of the treatment tool insertion/withdrawal device 62, to turn the power on to rotate the motor 36 in the direction of insertion. Accordingly, the roller 65 rotates in the normal direction, while the roller 66 rotates in the reverse direction, whereby the treatment tool insertion portion 21 is delivered into the treatment tool channel 7.

The reel 73 rotates as the treatment tool insertion portion 21 is drawn out. When the treatment section 22 provided at the distal end of the treatment tool 4 projects from the distal end of the endoscope 1, the operator operates the switch of the treatment tool insertion/withdrawal device 62 and cuts the power supply.

At this time, in the state at which the power supply of the treatment tool insertion/withdrawal device 62 is cut, the respective rollers 65, 66 can rotate freely, and hence the operator places his/her finger on the dial member 68 to rotate the dial member 68 in the normal direction. The roller 65 integrated with the dial member 68 rotates in the direction in which the treatment tool 4 is inserted, and consequently, the treatment tool 4 is moved forward with respect to the distal end of the endoscope 1 in proportion to the turning amount of the dial member 68.

On the other hand, when moving the treatment tool 4 backward, the operator rotates the dial member 68 in the direction opposite from the direction described above. The roller 65 rotates in the reverse direction according to the amount of turning of the dial member 68, and the treatment tool 4 moves backward with respect to the distal end of the endoscope 1.

When withdrawing the treatment tool 1, the operator operates the switch of the treatment tool insertion/withdrawal device 62, turns the power on, and rotates the motor 36 in the direction of withdrawal. Accordingly, the treatment tool 4 is withdrawn.

According to this embodiment, the rollers 65, 66 for causing the treatment tool 4 to be withdrawn electrically and automatically are partly exposed to the outside, so that the rollers 65, 66 can be rotated manually by working the dial member 68. Therefore, in the endoscope 1 in which the treatment tool 4 can be inserted and withdrawn electrically and automatically, the treatment section 22 projecting from the distal end of the endoscope 1 can be moved manually in the fore-and-aft direction with respect to the distal end of the endoscope 1 (or the subject portion). Therefore, the position of the treatment section 22 can be fine-adjusted to a position suitable for the treatment, and the treatment portion 22 can be finely pulled back.

Since parts of the rollers 65, 66 of the treatment tool insertion/withdrawal device 62 are configured to be a fine-adjustment mechanism, and the treatment tool 4 can be moved directly in the fore-and-aft direction, more reflexive operation is enabled by technician type personnel. In addition, since parts of rollers 65, 66 comprise the fine-adjustment mechanism, the structure of the device is simpler, and its size and cost are reduced.

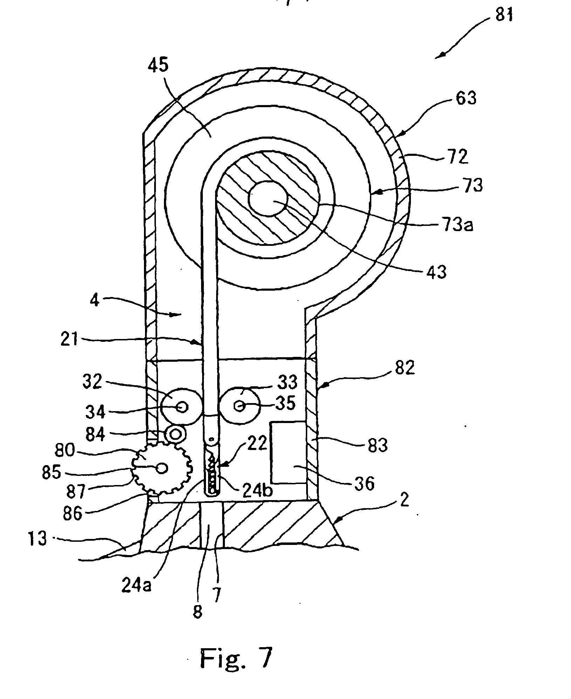

Referring to Fig. 7, a third embodiment is described, in which components appearing in the above-described embodiments are represented by the same reference numerals and the description of which is omitted.

As shown in Fig. 7, this embodiment is characterized in that the dial member 80 serving as the fine-adjustment mechanism is provided separately from the roller 32.

An endoscope 81 includes a treatment tool insertion/withdrawal device 82 which is mounted to the proximal end of the final operating element 2. The storage device 63 is located nearer the proximal end of the treatment tool insertion/withdrawal device 82.

The treatment tool insertion/withdrawal device 82 includes a roller 84 that rotates in conjunction with the roller 32, and a dial member 80 that rotates in conjunction with the roller 84 in a cover 83. The dial member 80 is rotatably supported by a revolving shaft 85 on the cover 83, and is partly exposed outwardly via an opening 86 formed on the side wall of the cover 83. In addition, a plurality of grooves 87 are formed on the outer peripheral surface of the dial member 80, so that the operator of the endoscope can place a finger thereon to rotate the dial member 80. The opening 86 is provided on the side wall on the side of the universal cable 13. Other structures of the treatment tool insertion/withdrawal device 82 are the same as those in the treatment tool insertion/withdrawal device 9 shown in Fig. 3.

In the endoscope 81, the roller 32 is not exposed toward the outside, but by rotating the dial member 80 manually, the roller 32 can be rotated in the direction of insertion, or in the direction of withdrawal.

Therefore, the same effects as in the second embodiment can be achieved. Since the ratio of the amount of rotation of the roller 32 with respect to the amount of rotation of the dial member 80 can be set to a predetermined value by the roller 84, the sensitivity of the fine-adjustment of the distal end position of the treatment tool 4 can be further fine-tuned.

The treatment tool insertion/withdrawal device 82 may be configured into a structure in which the roller 32 is rotated directly by the dial member 80 without the intermediary of the roller 84. It is also possible to provide a plurality of gears between the dial member 80 and the roller 32.

In the treatment tool insertion/withdrawal device 82, when the roller 32 is moved electrically, the dial member 80 is also driven. However, it is also possible to provide a transmission mechanism for mechanically connecting and disconnecting the roller 32 to/from the dial member 80 by a switch, not shown, between the roller 32 and the dial member 80.

The present invention is not limited to the above-described embodiments and may be applied widely.

For example, in the first embodiment, a dial member can be provided separately from the reel 42. In this case, the reel 42 is not exposed toward the outside and only the dial member is exposed toward the outside. When the dial member is rotated, the reel 42 is also driven correspondingly, and hence the treatment tool 4 can be moved in the fore-and-aft direction.

In Fig. 3, it is also possible to eliminate the dial member 47 and locate part of the outer peripheral surface of the flange 45 of the reel 42 so as to be exposed toward the outside, so that the operator can place his/her finger on the outer peripheral surface of the flange 45 to rotate the reel 42 directly by manual operation. Likewise, in Fig. 6, it is also possible to eliminate the dial 68 and locate part of the outer peripheral surface of the roller 65 so as to be exposed toward the outside, so that the operator can place his/her finger on the outer peripheral surface of the roller 65 to manually and directly rotate the roller 65. In these cases, movement of the treatment tool in the fore-and-aft direction is enabled with a simple structure. In this case, the reel 42 or the roller 65 serves as the fine-adjustment mechanism.

The fine-adjustment mechanism may be configured of a lever for rotating the reel 42 or the roller 65 by its reciprocal movement operating a ratchet mechanism, instead of the dial members 47, 68 having a rotationally symmetric shape.

The final operating elements for the treatment tool insertion/withdrawal devices 9, 62, 82 are not limited to the switch, and may be a plurality of buttons and the like.

Although examples of the endoscope for medical use have been described in the above-described embodiments, the present invention may also be applicable to the endoscope for industrial use. The endoscope may be a flexible scope or a rigid scope.

While there has been shown and described what are considered to be preferred embodiments of the invention, it will, of course, be understood that various modifications and changes in form or detail could readily be made without departing from the scope of the invention. It is therefore intended that the invention not be limited to the exact forms described and illustrated, but constructed to cover all modifications that may fall within the scope of the appended claims.

## Claims

1. An endoscope comprising:
an insertion portion (3) at least partly insertable into a cavity and having a treatment tool channel (7) enabling passage therethrough of a treatment tool (4),
a rotating reel (42) for winding thereon the treatment tool; and
an insertion/withdrawal mechanism (42, 43, 32, 33, 65, 66) for inserting and withdrawing the treatment tool (4) into and from the treatment tool channel (7), the insertion withdrawal mechanism including:
a plurality of rollers (32, 33, 65, 66) configured to grasp the treatment tool (4); and
a motor (36) configured to drive at least one of the rollers (32, 33, 65, 66) to move the treatment tool in the fore-and-aft direction with respect to the treatment tool channel,
**characterized in that** the endoscope further comprises,
a mechanical fine-adjustment mechanism (47, 48, 68, 69, 80, 84, 87) for fine-adjusting the position of a distal end of the treatment tool (4) relative to a distal end of the treatment tool channel, the fine-adjustment mechanism adapted to be manually operated to move the treatment tool in the fore-and-aft direction with respect to the treatment tool channel by rotating one of:
the reel (42); and
at least one of the rollers (32, 33, 65, 66).

2. An endoscope according to Claim 1, wherein the mechanical fine-adjustment mechanism (47) comprises a mechanism for rotating the reel by which the treatment tool (4) can be reeled from and into the channel (7).

3. An endoscope according to Claim 1, wherein the mechanical fine-adjustment mechanism (68) is operative to rotate at least one of the rollers.

4. An endoscope according to one of Claims 1 to 3, wherein the insertion portion (3) has a flexible body, and including an operating element (2, 12) for changing the position of the distal end of the insertion portion.

5. An endoscope according to one of Claims 1 to 4, further comprising a storage section (10) comprising the reel (42) for winding and storing the treatment tool in the endoscope.

6. An endoscope according to Claim 2, wherein the mechanical fine-adjustment mechanism includes a dial (47) that rotates with the reel.

7. An endoscope according to Claim 3, wherein the mechanical fine-adjustment mechanism includes a dial (68) that rotates with the rollers (32, 33).

8. An endoscope according to Claim 3, wherein the mechanical fine-adjustment mechanism includes a dial (80) that is moved in conjunction with the rollers (32, 33).

9. An endoscope according to Claim 7,wherein the dial (68) is mechanically coupled to manually rotate at least one of the rollers (65, 66).

10. An endoscope according to one of Claims 1 to 9, including a control configured for disengaging the motor (36)to allow the rollers to turn freely in a mode in which the mechanical fine-adjustment mechanism is used.

11. An endoscope according to Claim 7, wherein the dial (68) has a center of rotation which is co-extensive with a center of rotation of one of the rollers (65, 66).

12. An endoscope according to Claim 8, wherein the dial (80) is coupled to at least one of the rollers (32, 33) via at least one gear (84).

13. An endoscope according to one of Claims 1 to 12, wherein a peripheral surface of the dial (47, 68, 80) is grooved to facilitate the rotation thereof.

14. An endoscope according to one of Claims 1 to 13, wherein the treatment tool includes a cap adjacent a distal end thereof, the cap being instrumental to serve as a stop which prevents the distal end of the treatment tool from passing between the rollers.

15. An endoscope according to one of Claims 1 to 13, further including an auxiliary tool opening into the treatment tool channel, the opening being located below the insertion/withdrawal mechanism to enable insertion of another tool into the treatment channel when the treatment tool has been withdrawn past the auxiliary tool opening.

16. An endoscope according to one of Claims 1 to 13, further including a grasping tool provided at the distal end of the treatment tool and a manual controller therefor.

## Patentansprüche

1. Endoskop, aufweisend:
einen Einführabschnitt (3), der zumindest teilweise in einen Hohlraum eingeführt werden kann und einen Behandlungswerkzeugkanal (7) hat, durch den ein Behandlungswerkzeug (4) geführt werden kann,
eine rotierende Spule (42) zum Aufspulen des Behandlungswerkzeugs darauf; und
einen Einführ-/Rückzugsmechanismus (42, 43, 32, 33, 65, 66) zum Einführen und Zurückziehen des Behandlungswerkzeugs (4) in den und aus dem Behandlungswerkzeugkanal (7), wobei der Einführ-/Rückzugsmechanismus enthält:
eine Mehrzahl Rollen (32, 33, 65, 66), die zum Greifen des Behandlungswerkzeugs (4) konfiguriert sind; und
einen Motor (36), der zum Antreiben mindestens einer der Rollen (32, 33, 65, 66) konfiguriert ist, um das Behandlungswerkzeug bezüglich des
Behandlungswerkzeugkanals in Vorwärts- und Rückwärtsrichtung zu bewegen, **dadurch gekennzeichnet, dass** das Endoskop ferner aufweist:
einen mechanischen Feinstellmechanismus (47, 48, 68, 69, 80, 84, 87) zum Feineinstellen der Position des distalen Endes des Behandlungswerkzeugs (4) relativ zum distalen Ende des Behandlungswerkzeugkanals, wobei der Feinstellmechanismus zur manuellen Betätigung eingerichtet ist, um das Behandlungswerkzeug bezüglich des Behandlungswerkzeugkanals in Vorwärts- und Rückwärtsrichtung zu bewegen,
indem entweder
die Spule (42); oder
mindestens eine der Rollen (32, 33, 65, 66)
gedreht wird.

2. Endoskop nach Anspruch 1, bei dem der mechanische Feinstellmechanismus (47) einen Mechanismus zum Drehen der Spule aufweist, so dass das Behandlungswerkzeug (4) aus dem Kanal (7) gezogen und in den Kanal eingebracht werden kann.

3. Endoskop nach Anspruch 1, bei dem der mechanische Feinstellmechanismus (68) wirksam ist, um mindestens eine der Rollen zu drehen.

4. Endoskop nach einem der Ansprüche 1 bis 3, bei dem der Einführabschnitt (3) einen flexiblen Körper hat und ein Betätigungselement (2, 12) zum Ändern der Position des distalen Endes des Einführabschnitts enthält.

5. Endoskop nach einem der Ansprüche 1 bis 4, das ferner einen die Spule (42) aufnehmenden Aufbewahrungsabschnitt (10) zum Aufspulen und Aufbewahren des Behandlungswerkzeugs im Endoskop aufweist.

6. Endoskop nach Anspruch 2, bei dem der mechanische Feinstellmechanismus eine Skalenscheibe (47) enthält, die sich mit der Spule dreht.

7. Endoskop nach Anspruch 3, bei dem der mechanische Feinstellmechanismus eine Skalenscheibe (68) enthält, die sich mit den Rollen (32, 33) dreht.

8. Endoskop nach Anspruch 3, bei dem der mechanische Feinstellmechanismus eine Skalenscheibe (80) enthält, die gemeinsam mit den Rollen (32, 33) bewegt wird.

9. Endoskop nach Anspruch 7, bei dem die Skalenscheibe (68) mechanisch so gekoppelt ist, um mindestens eine der Rollen (32, 33) manuell zu drehen.

10. Endoskop nach einem der Ansprüche 1 bis 9, mit einer Steuerung, die zum Entkoppeln des Motors (36) konfiguriert ist, damit sich die Rollen in einem Modus frei drehen können, in dem der mechanische Feinstellmechanismus verwendet wird.

11. Endoskop nach Anspruch 7, bei dem die Skalenscheibe (68) einen Rotationsmittelpunkt hat, der mit dem Rotationsmittelpunkt einer der Rollen (65, 66) zusammenfällt.

12. Endoskop nach Anspruch 8, bei dem die Skalenscheibe (80) mit mindestens einer der Rollen (32, 33) über mindestens ein Zahnrad (84) gekoppelt ist.

13. Endoskop nach einem der Ansprüche 1 bis 12, bei dem die Außenumfangsfläche der Skalenscheibe (47, 68, 80) gerillt ist, um ihre Drehung zu erleichtern.

14. Endoskop nach einem der Ansprüche 1 bis 13, bei dem das Behandlungswerkzeug eine Kappe neben seinem distalen Ende enthält, wobei die Kappe die Aufgabe hat, als Anschlag zu dienen, der verhindert, dass sich das distale Ende des Behandlungswerkzeugs zwischen den Rollen hindurchbewegt.

15. Endoskop nach einem der Ansprüche 1 bis 13, ferner mit einer Hilfswerkzeugöffnung, die in den Behandlungswerkzeugkanal mündet, wobei die Öffnung unterhalb des Einführ-/Rückzugsmechanismus angeordnet ist, um das Einführen eines anderen Werkzeugs in den Behandlungskanal zu ermöglichen, wenn das Behandlungswerkzeug an der Hilfswerkzeugöffnung vorbei zurückgezogen worden ist.

16. Endoskop nach einem der Ansprüche 1 bis 13, ferner mit einem Greifwerkzeug am distalen Ende des Behandlungswerkzeugs und einem manuellen Bedienungselement dafür.

## Revendications

1. Endoscope comprenant :
une partie d'insertion (3) pouvant être insérée au moins partiellement dans une cavité et comportant un canal d'outil de traitement (7) permettant le passage à travers celle-ci de l'outil de traitement (4),
une bobine rotative (42) destinée à enrouler sur celle-ci l'outil de traitement,
et
un mécanisme d'insertion/rétractation (42, 43, 32, 33, 65, 66) destiné à insérer et à rétracter l'outil de traitement (4) dans et depuis le canal d'outil de traitement (7), le mécanisme d'insertion/rétractation comprenant :
une pluralité de rouleaux (32, 33, 65, 66) configurés pour saisir l'outil de traitement (4) ; et
un moteur (36) configuré pour entraîner au moins l'un des rouleaux (32, 33, 65, 66) pour déplacer l'outil de traitement dans le sens de la longueur par rapport au canal d'outil de traitement,
**caractérisé en ce que** l'endoscope comprend en outre
un mécanisme de réglage micrométrique mécanique (47, 48, 68, 69, 80, 84, 87) destiné à régler de façon micrométrique la position d'une extrémité distale de l'outil de traitement (4) par rapport à une extrémité distale du canal d'outil de traitement, le mécanisme de réglage micrométrique étant adapté pour être actionné manuellement pour déplacer l'outil de traitement dans le sens de la longueur par rapport au canal d'outil de traitement en faisant tourner l'un parmi :
la bobine (42) ; et
au moins l'un des rouleaux (32, 33, 65, 66).

2. Endoscope selon la revendication 1, dans lequel le mécanisme de réglage micrométrique mécanique (47) comprend un mécanisme destiné à faire tourner la bobine par laquelle l'outil de traitement (4) peut être enroulé et déroulé dans le canal (7).

3. Endoscope selon la revendication 1, dans lequel le mécanisme de réglage micrométrique (68) est opérationnel pour faire tourner au moins l'un des rouleaux.

4. Endoscope selon l'une quelconque des revendications 1 à 3, dans lequel la partie d'insertion (3) possède un corps souple, et comprenant un élément d'actionnement (2, 12) destiné à changer la position de l'extrémité distale de la partie d'insertion.

5. Endoscope selon l'une quelconque des revendications 1 à 4, comprenant en outre une section de stockage (10) comprenant la bobine (42) destinée à enrouler et à stocker l'outil de traitement dans l'endoscope.

6. Endoscope selon la revendication 2, dans lequel le mécanisme de réglage micrométrique mécanique comprend un cadran (47) qui tourne avec la bobine.

7. Endoscope selon la revendication 3, dans lequel le mécanisme de réglage micrométrique mécanique comprend un cadran (68) qui tourne avec les rouleaux (32, 33).

8. Endoscope selon la revendication 3, dans lequel le mécanisme de réglage micrométrique mécanique comprend un cadran (80) qui est déplacé en liaison avec les rouleaux (32, 33).

9. Endoscope selon la revendication 7, dans lequel le cadran (68) est mécaniquement couplé pour faire tourner manuellement au moins l'un des rouleaux (65, 66).

10. Endoscope selon l'une quelconque des revendications 1 à 9, comprenant une commande configurée pour désengrener le moteur (36) pour permettre aux rouleaux de tourner librement dans un mode dans lequel le mécanisme de réglage micrométrique mécanique est utilisé.

11. Endoscope selon la revendication 7, dans lequel le cadran (68) possède un centre de rotation qui est coextensif à un centre de rotation de l'un des rouleaux (65, 66).

12. Endoscope selon la revendication 8, dans lequel le cadran (80) est couplé à au moins l'un des rouleaux (32, 33) via au moins un engrenage (84).

13. Endoscope selon l'une quelconque des revendications 1 à 12, dans lequel une surface périphérique du cadran (47, 68, 80) est rainurée pour faciliter la rotation de celui-ci.

14. Endoscope selon l'une quelconque des revendications 1 à 13, dans lequel l'outil de traitement comprend un capuchon adjacent à une extrémité distale de celui-ci, le capuchon contribuant à servir de butée qui empêche l'extrémité distale de l'outil de traitement de passer entre les rouleaux.

15. Endoscope selon l'une quelconque des revendications 1 à 13, comprenant en outre une ouverture d'outil auxiliaire dans le canal d'outil de traitement, l'ouverture étant placée au-dessous du mécanisme d'insertion/rétractation pour permettre l'insertion d'un autre outil dans le canal de traitement lorsque l'outil de traitement a été rétracté au-delà de l'ouverture d'outil auxiliaire.

16. Endoscope selon l'une quelconque des revendications 1 à 13, comprenant en outre un outil de saisie prévu au niveau de l'extrémité distale de l'outil de traitement et un contrôleur manuel pour celui-ci.
